# EUROPEAN PATENT APPLICATION

(11) **EP 3 995 169 A1**
(43) Date of publication of application: **11.05.2022**
(21) Application number: 20848033.5
(22) Date of filing: 19.06.2020
(51) Int. Cl.: A61M 25/00, A61M 25/098

(54) **CATHETER**

(30) Priority: 31.07.2019 JP 2019140688
(71) Applicant: TERUMO Kabushiki Kaisha, Tokyo 151-0072 (JP)
(72) Inventor: HAMURO, Kouta, Fujinomiya-shi, Shizuoka 418-0015 (JP); FUKUOKA, Tetsuya, Ashigarakami-gun, Kanagawa 259-0151 (JP)
(74) Representative: Casalonga
(86) International application number: PCT/JP2020/024079
(87) International publication number: WO 2021/019945

(57) **Abstract**

Provided is a catheter that is excellent in visibility of a braid distal end portion inside and outside the body, is excellent in stenosed site penetrability and trackability to a tortuous blood vessel, and prevents the braid from spreading out due to the stenosed site penetration. The catheter includes a shaft (100), the shaft (100) includes an inner layer (12), an outer layer (11), and a braid (6) that is disposed between the inner layer (12) and the outer layer (11) and includes a plurality of metal wires being woven together, the braid (6) includes a welding portion (10) at a further proximal side than a distal end (3) of the shaft and in the vicinity of a distal end of the braid (6), and the shaft (100) includes a radiopaque marker (7) at a position separated from the welding portion (10), thereby preventing the braid (6) from spreading out due to buckling of the shaft (100), and improving the blood vessel trackability.

## Description

### Technical Field

The present invention relates to a catheter.

### Background Art

A braid in which metal wires are woven together is typically embedded into a catheter for kink prevention, and a braid end portion in the vicinity of a distal end of the catheter is fixed with resin of an outer layer or an inner layer or a distal tip so as not to prevent damage of a vascular wall.

In recent years, in some catheters, intersection points at the braid end portion are welded by a laser, thereby fixing the braid end portion.

### Citation List

### Patent Literature

PTL 1: US Patent No. 2008/0125752
PTL 2: JP-A-2014-144163

### Summary of Invention

### Technical Problem

PTL 1 discloses a catheter in which a braid end portion is welded. Moreover, PTL 2 discloses a catheter in which a distal tip is provided at a distal end of a shaft, and in order to prevent the distal tip from pulling out from the shaft and falling off, a bulging portion larger than the thickness of the metal wire is formed in the braid end portion.

However, in this method, an interface of the resin is present between the distal tip and the shaft, and the thickness of the outer layer of the bulging portion is reduced, so that the tensile strength may decreased.

Moreover, even the distal tip using the resin containing an X-ray contrast agent becomes thin-walled when the catheter is thin, so that it is difficult to visually recognize the distal tip under the X-ray contrast.

The distal end position of the braid cannot be recognized in such a state, so that the distal tip of the catheter may be brought into contact with a vascular wall to cause separation of the vascular wall.

### Solution to Problem

The invention below attains the above objects.

(1) A catheter according to the invention is a catheter including a shaft including a distal end and a proximal end, in which the shaft includes an inner layer, an outer layer, and a braid that is disposed between the inner layer and the outer layer and includes a plurality of metal wires that are woven together, the braid includes a welding portion at a further proximal side than the distal end of the shaft that welds intersection points of the metal wires in a vicinity of a distal end of the braid, and the shaft includes a radiopaque marker at a position separated from the welding portion to the proximal side.
(2) In the catheter according to the above (1), the radiopaque marker may have a length in a longitudinal axis direction of the shaft, and the length in the longitudinal axis direction of the radiopaque marker may be longer than a distance from the welding portion to a distal end of the radiopaque marker.
(3) In the catheter according to the above (1) or (2), the radiopaque marker may be a coil wire, and a diameter of the coil wire may be less than a distance from a distal end of the welding portion to the distal end of the radiopaque marker.

### Advantageous Effect of Invention

With the catheter according to the invention, the welding portion of the intersection points that is located at the distal side of the braid is located at the further distal side than the distal end of the radiopaque marker, so that the position of the welding portion can be visually observed outside the body, and the radiopaque marker of the catheter inside the body can be visually recognized under the X-ray contrast. Therefore, the catheter is easily inspected when being manufactured, and can be accurately positioned when being moved forward in the unit of millimeters during surgery.

In addition, a distal end of the radiopaque marker is separated from the welding portion, so that the distal end portion of the catheter becomes more flexible to improve the trackability and the branch selectivity in a complicated and thin blood vessel such as a hepatic artery.

Accordingly, the catheter easily reaches a lesion area to shorten the surgery time, thereby allowing the reduction in the burden of a patient, and the reduction in labor costs.

### Brief Description of Drawings

[Fig. 1] Fig. 1 is a cross-sectional view of a distal end portion of a catheter according to a first embodiment of the invention.
[Fig. 2] Fig. 2 is a cross-sectional view of a distal end portion of a catheter according to a second embodiment of the invention.

### Description of Embodiments

Hereinafter, preferred embodiments of the invention are provided, and are described in details with reference to the accompanying drawings. Note that, the size ratios in the drawings may be exaggerated for convenience of explanation, and may be different from the actual ratios in some cases. In the present specification, a side of a catheter to be inserted into a living body lumen is referred to as a "distal side", and a side at which the catheter is operated is referred to as a "proximal side".

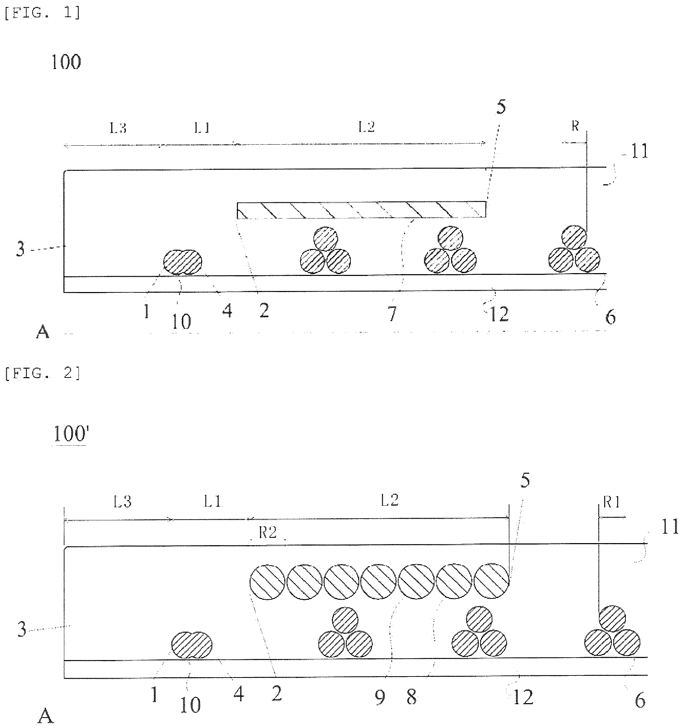

A shaft 100 of a catheter as a first embodiment illustrated in Fig. 1 includes an outer layer 11 and an inner layer 12, and includes a braid 6 therebetween in which metal wires and the like are woven together.

A plurality of metal wires are woven together to form the braid 6 in a tubular shape so as to have gaps penetrating between an inner peripheral surface and an outer peripheral surface. The braid 6 includes a welding portion 10 formed in such a manner that intersection points of the metal wires mutually crossing are welded.

This is for preventing the braid 6 from spreading out because the shaft 100 is buckled when a shaft distal end 3 is brought into contact with a hard stenosed site or the like, and a part of the metal wires from breaking through the outer layer 11 of the shaft 100.

The welding portion 10 is preferably positioned on a distal end of the braid 6, but may be welded on a position at a further proximal side than the distal end of the braid and at a further distal side than a distal end of an radiopaque marker, so as to be more firmly fixed.

Alternatively, in order to reduce a level difference in physical properties between the welding portion 10 and the shaft distal end 3, the metal wires may protrude from the welding portion 10 to the distal side.

The welding portion 10 includes a welding portion distal end 1 and a welding portion proximal end 4. In the present embodiment, the braid 6, one of which is a single wire and the other of which is a multiple wire including two metal wires, is formed in such a manner that the single wire and the multiple wire are crossed and woven together. Note that, single wires or multiple wires may be woven together to form the braid 6. Moreover, metal wires having different thicknesses and types may be woven together to form the braid 6.

Examples of materials for the braid 6 may include tungsten or stainless steel wire. The metal wire in the braid 6 may have an arbitrary thickness, and preferably has a diameter of 5 to 100 µm, preferably 15 to 60 µm. The cross-section of the metal wire in the braid 6 is circular, and may be elliptical, rectangular, or oval.

In the first embodiment illustrated in Fig. 1, the welding portion distal end 1 is disposed at the proximal side that is distant by a distance L3 from the shaft distal end 3. In addition, a distal end 2 of a tubular marker 7 serving as an radiopaque marker is disposed at a position that is distant by a distance L1 from the welding portion distal end 1 to the proximal side. The distance L1 is less than a distance between the distal end 2 and a proximal end 5 of the tubular marker 7, in other words, a length L2 in a longitudinal axis direction of the tubular marker 7. Note that, the longitudinal axis direction is a direction along the longitudinal axis extending between a distal end and a proximal end of the shaft 100, for example, a direction along a central axis of a lumen of the shaft 100.

Therefore, even when the shaft distal end 3 is brought into contact with a stenosed site, the braid distal end does not spread out between the welding portion distal end 1 and the distal end 2 of the tubular marker 7 by the force transmitted from the shaft distal end 3, and the force that is transmitted from the shaft distal end 3 is transmitted to the tubular marker 7 with high efficiency. Therefore, the shaft 100 can pass through the stenosed site without buckling.

The tubular marker 7 (radiopaque marker) is disposed at a position separated from the welding portion 10 to the proximal side. Therefore, a position of the welding portion 10 can be visually observed through a transparent material outside the body, and the tubular marker 7 of the catheter inside the body can also be visually recognized under the X-ray contrast. Therefore, the catheter is easily inspected when being manufactured, and can be accurately positioned when being moved forward in the unit of millimeters during surgery. In addition, a distal end of the tubular marker 7 is separated from the welding portion 10, so that the distal end portion of the catheter becomes more flexible to improve the trackability and the branch selectivity in a complicated and thin blood vessel such as a hepatic artery. Accordingly, the catheter easily reaches a lesion area to shorten the surgery time, thereby allowing the reduction in the burden of a patient, and the reduction in labor costs.

The tubular marker 7 having a cylindrical shape is formed by using an iridium alloy and the like. The thickness of the tubular marker 7 is 5 to 100 µm, preferably 15 to 60 µm. The length in the longitudinal axis direction of the tubular marker 7 is 0.5 to 10 mm, preferably 1 to 3 mm. The tubular marker 7 may be fitted into the shaft 100 or embedded into the shaft 100 with a resin.

A shaft 100' according to a second embodiment illustrated in Fig. 2 includes a coil marker 9 as the radiopaque marker in which a coil wire 8 made of metal that is recognizable under radiography is wound, and is common to that in the first embodiment other than the coil marker 9, so that an explanation thereof is omitted.

A distal end 2 of the coil marker 9 is disposed at a position distant by a distance L1 from the welding portion distal end 1 to the proximal side, and the distance L1 is less than a length L2 in the longitudinal axis direction of the coil wire 8.

The coil marker 9 is flexible and easily deforms, so that a distal end portion of the shaft 100' has improved trackability to the bent blood vessel.

The coil marker 9 is formed by using a gold alloy and the like. A diameter R2 of the coil wire 8 that forms the coil marker 9 is 5 to 100 µm, preferably 15 to 60 µm. The coil wire 8 is coiled around the shaft 100. The coil wire 8 is wound such that a length in the longitudinal axis direction of the coil marker 9 is 0.5 to 10 mm, preferably 1 to 3 mm. The diameter R2 of the coil wire 8 is not specially limited, but is preferably less than the distance L1 from the welding portion distal end 1 to the distal end 2 of the coil marker 9 (radiopaque marker). This prevents the coil wire 8 from becoming too thick, and the coil marker 9 is flexible and easily deforms, so that a distal end portion of the shaft 100' has improved trackability to the bent blood vessel.

Therefore, the shaft 100' can pass through a bifurcated blood vessel of the coronary artery and a tortuous blood vessel or a branched blood vessel of the hepatic artery by following the guide wire.

In the above, the invention has been described with the preferred embodiments, however, the invention is not limited to the embodiments, and it is needless to say that various modifications are possible without deviating from the scope of the invention.

Note that, the application is based upon and claims the benefit of priority of the prior Japanese Patent Application No. 2019-140688, filed on July 31, 2019, the entire contents of which are incorporated herein by reference.

### Reference Signs List

1 welding portion distal end
2, 2' marker distal end
3 shaft distal end
4 welding portion proximal end
5, 5' marker proximal end
6 braid
7 tubular marker
8 coil wire
9 coil marker
10 welding portion
11 outer layer
12 inner layer
100 shaft
L1 distance from welding portion distal end to marker distal end
L2 length of marker
L3 length from shaft distal end to welding portion distal end
R, R1 diameter of metal wire in braid
R2 coil wire diameter

## Claims

1. A catheter comprising a shaft including a distal end and a proximal end, wherein
the shaft includes an inner layer, an outer layer, and a braid that is disposed between the inner layer and the outer layer and includes a plurality of metal wires that are woven together,
the braid includes a welding portion at a further proximal side than the distal end of the shaft that welds intersection points of the metal wires in a vicinity of a distal end of the braid, and
the shaft includes a radiopaque marker at a position separated from the welding portion to the proximal side.

2. The catheter according to claim 1, wherein the radiopaque marker has a length in a longitudinal axis direction of the shaft, and the length in the longitudinal axis direction of the radiopaque marker is longer than a distance from the welding portion to a distal end of the radiopaque marker.

3. The catheter according to claim 1 or 2, wherein the radiopaque marker is a coil wire, and a diameter of the coil wire is less than a distance from a distal end of the welding portion to the distal end of the radiopaque marker.
